# FASCICULE DE BREVET EUROPEEN

(11) **EP 2 726 515 B1**
(45) Date de publication et mention de la délivrance du brevet: **01.04.2015**
(21) Numéro de dépôt: 12738539.1
(22) Date de dépôt: 27.06.2012
(51) Int. Cl.: C08F 8/26, C08F 8/42, C07D 257/04, C07D 403/00

(54) **REACTIF ORGANOSTANNIQUE ALCOXYLE SUPPORTE, PREPARATION ET UTILISATION POUR LA SYNTHESE DE TETRAZOLES EN PHASE HETEROGENE**
GETRÄGERTER ALKOXYLIERTER ORGANOTINREAKTAND, HERSTELLUNG UND VERWENDUNG ZUR SYNTHESE VON TETRAZOLEN IN DER HETEROGENEN PHASE
SUPPORTED ALKOXYLATED ORGANOTIN REACTANT, PREPARATION AND USE FOR HETEROGENEOUS-PHASE SYNTHESIS OF TETRAZOLES

(30) Priorité: 30.06.2011 FR 1102058
(43) Date de publication de la demande: 07.05.2014
(73) Titulaire: Centre National de la Recherche Scientifique, 75016 Paris (FR); Universite de Nantes, 44000 Nantes (FR)
(72) Inventeur: LE GROGNEC, Erwan, F-56100 Lorient (FR); QUINTARD, Jean-Paul, F-44240 La Chapelle sur Erdre (FR); KERRIC, Gaelle, F-56700 Merlevenez (FR); CHRETIEN, Jean-Mathieu, F-53210 Argentre (FR); ZAMMATTIO, Françoise, F-44220 Coueron (FR)
(74) Mandataire: Goulard, Sophie
(86) Numéro de dépôt international: PCT/FR2012/051480
(87) Numéro de publication internationale: WO 2013/001235

(56) Documents cités:
- CHRÉTIEN J.M AT AL.: "Preparation of Allyltin Reagents Graftedon SolidSupport: Clean andEasilyRecyclable Reagents for Allylation of Aldehydes", CHEMISTRY - A EUROPEAN JOURNAL, vol. 12, 6 juin 2006 (2006-06-06), pages 6816-6828, XP002673536, DOI: 10.1002/chem.200501595
- KERRIC G. ET AL.: "Synthesis, characterization and primary evaluation of the synthetic efficiency of supported vinyltins and allyltins", JOURNAL OF ORGANOMETALLIC CHEMISTRY, vol. 695, no. 9, 1 mai 2010 (2010-05-01), pages 1414-1424, XP002673537,
- CURRAN ET AL.: "tris(2-perfluorohexylethyl)tin azide: A new reagent for preparation of 5-substituted tetrazoles from nitriles with purification by fluorous/organic liquid-liquid extraction", TETRAHEDRON, vol. 55, no. 29, 16 juillet 1999 (1999-07-16), pages 8997-9006, XP002673538,

## Description

La présente invention est relative à un réactif organostannique alcoxylé supporté, à son procédé de préparation, à l'utilisation d'un tel réactif en tant que catalyseur pour la synthèse organique en phase hétérogène, ainsi qu'à un procédé de synthèse de tétrazoles 5-substitués ou 1,5-disubstitués en phase hétérogène, mettant en oeuvre un tel réactif.

La fonction tétrazole est une fonction chimique que l'on retrouve dans de nombreuses molécules ayant des applications dans des domaines variés tels que le domaine des explosifs, de la photographie, de l'agrochimie, et de la pharmacie. Dans le domaine pharmaceutique, on peut en particulier citer à titre d'exemple, la famille des sartans qui sont des antagonistes de l'angiotensine II, utilisés pour le traitement des maladies cardiovasculaires ainsi que les inhibiteurs de la cyclooxygénase 2 (COX-2) qui sont des anti-inflammatoires.

Il est essentiel pour les industriels produisant et commercialisant des molécules ayant une ou des fonctions tétrazole, et notamment pour les laboratoires pharmaceutiques, de pouvoir les synthétiser de manière efficace, sûre (pour les personnels et pour l'environnement), à un prix de revient acceptable et avec des puretés les plus importantes possibles notamment vis-à-vis des teneurs résiduelles en métaux.

La synthèse de composés à fonction tétrazole peut se faire selon différents procédés. Il est par exemple possible de mettre en oeuvre des procédés faisant intervenir de l'acide azothydrique (HN₃) qui est cependant un gaz toxique et explosif dangereux à manipuler. Il est aussi possible de mettre en oeuvre des procédés faisant intervenir un azoture métallique (Na, Al, B, Si, ...) qui réagit avec un nitrile correspondant au tétrazole que l'on souhaite obtenir, éventuellement en présence d'un acide de Lewis. C'est ainsi qu'il a déjà été proposé, notamment dans la demande de brevet EP-A1-0 708 103, un procédé de synthèse d'un dérivé ayant une fonction tétrazole par réaction d'un nitrile correspondant avec un azoture alcalin tel que l'azoture de sodium et du chlorhydrate de triéthylamine. Dans ce cas, la synthèse a lieu en phase liquide et des étapes de purification supplémentaires, par exemple par cristallisation sont nécessaires pour isoler le dérivé ayant une fonction tétrazole attendu, ce qui entraîne des surcoûts dé production. Il est également possible, de synthétiser des molécules comportant une fonction tétrazole en utilisant des dérivés organostanniques tels que des azotures de trialkyl- ou de triarylétain comme décrit par exemple dans le brevet US 5,399,578. Cependant si l'utilisation d'azotures organostanniques permet de synthétiser de manière efficace des molécules comportant une fonction tétrazole, son intérêt est cependant contrebalancé par la nécessité d'effectuer des purifications difficiles de façon à séparer complètement les résidus stanniques des tétrazoles synthétisés, ce qui entraîne également des surcoûts de synthèse.

Il existe donc un besoin pour un réactif utilisable en tant que catalyseur et qui permette notamment de synthétiser des composés à fonction tétrazole selon un procédé qui soit à la fois efficace et économique, en particulier selon un procédé dans lequel la récupération des produits synthétisés soit facilitée tout en évitant au maximum leur contamination par des résidus stanniques.

La présente invention a donc pour objet un réactif organostannique alcoxylé de formule (I) suivante : 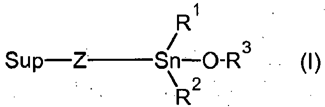 dans laquelle :
- Sup représente un support solide macromoléculaire, de préférence insoluble dans les solvants organiques ;
- Z représente un bras de liaison entre le support solide macromoléculaire Sup et l'atome d'étain, Z étant choisi parmi les groupements de formules (Z-1) à (Z-6) suivantes :

   -(CH₂)ₙ- (Z-1)

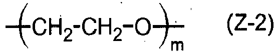

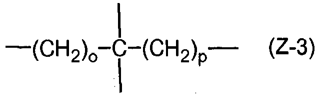

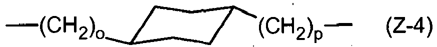

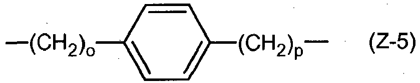

   -CH₂-O-(CH₂)_{q}-CH₂-CH₂- (Z-6)
dans lesquelles :
- n et m, indépendamment l'un de l'autre, sont des nombres entiers variant de 2 à 24, et de préférence de 3 à 12,
- o et p, indépendamment l'un de l'autre, sont des nombres entiers variant de 1 à 24, et de préférence de 1 à 12
- q est un nombre entier variant de 1 à 24 et de préférence de 1 à 2,
- R¹, R² et R³, indépendamment les uns des autres, représentent un radical alkyle linéaire en C₁-C₂₄, un radical alkyle ramifié ou cyclique en C₃-C₂₄, alcényle linéaire en C₂-C₂₄, alcényle ramifié ou cyclique en C₃-C₂₄, aryle ou arylalkyle.

Le polymère du support solide peut être choisi parmi les polymères à base de styrène tels que les polystyrènes, les polystyrènes réticulés, les copolymères butadiène/styrène, acrylonitrile/butadiène/styrène ou méthacrylate de méthyle/butadiène/styrène, les poly(phénylène éthers), les poly(phénylène sulfurés), et les polyamides.

Il a été mis en évidence que des polystyrènes réticulés sont particulièrement indiqués comme support pour les catalyseurs hétérogènes de la présente invention. Dans ce cas, les polystyrènes peuvent être obtenus par copolymérisation du styrène et d'un agent de réticulation. A titre d'agent réticulant, on peut notamment citer par exemple le méthacrylate d'éthane-1,2-diol, le méthacrylate de propane-1,2,3-triol, le butadiène, le trivinylbenzène et le divinyltoluène, le divinylbenzène, ce dernier étant particulièrement préféré selon l'invention.

Ledit support polymère est de préférence insoluble dans les solvants organiques utilisés en synthèse organique et notamment pour la synthèse des tétrazoles. Un tel support est néanmoins capable d'interagir avec ledit solvant, en particulier, ces polymères, qu'ils soient macroporeux ou microporeux, gonflent plus ou moins selon la nature du solvant utilisé.

Un support polymère particulièrement préféré selon l'invention est choisi parmi les supports polymères résultant de la copolymérisation du styrène et du divinylbenzène en tant qu'agent réticulant.

Parmi les radicaux alkyle mentionnés pour R¹, R², R³, les radicaux correspondent de préférence à méthyle, éthyle, *n*-propyle, *iso*-propyle, *n*-butyle, 2-butyle, *iso*-butyle, *tert*-butyle, *n*-pentyle, *iso*-pentyle, *néo*-pentyle, *tert*-pentyle, hexyle, *n*-octyle, *iso*-octyle, 2-éthyl-1-hexyle, 2,2,4-triméthylpentyle, nonyle, décyle, dodécyle, cyclopentyle, cyclohexyle, cycloheptyle, méthylcyclohexyle, Les substituants les plus favorables étant méthyle, éthyle, n-propyle, iso-propyle et *n-*butyle.

Parmi les radicaux alcényle mentionnés ci-dessus pour R¹, R², et R³, on peut en particulier citer les radicaux vinyle, 1-propényle, 2-propényle et butényle.

Les radicaux aryle mentionnés ci-dessus pour R¹, R² et R³, correspondent à un groupement hydrocarboné aromatique monocyclique ou polycyclique éventuellement mono ou polysubstitué. A titre de radical aryle, on peut en particulier citer les groupements naphtyle, anthranyle, phénantryle, o-tolyle, p-tolyle, xylyle, éthylphényle, mésityle, phényle et benzyle.

Selon une forme de réalisation préférée de l'invention, les réactifs de formule (I) sont choisis parmi les composés dans lesquels :
- Sup est un support solide polymère à base de styrène réticulé ou non ;
- Z = -(CH₂)ₙ avec n = 3 ou 4 ; et
- les radicaux R¹, R², et R³ ont les significations suivantes :
   i) R¹ = R² = R³ = méthyle, éthyle, propyle ou butyle ;
   ii) R¹ = R² = méthyle et R³ = éthyle, propyle ou butyle ;
   iii) R¹ = R² = éthyle et R³ = méthyle, propyle ou butyle ;
   iv) R¹ = R² = propyle et R³ = méthyle, éthyle ou butyle ; ou
   v) R¹ = R² = butyle et R³ = méthyle, propyle ou éthyle.

La présente invention a également pour objet un procédé de préparation d'un réactif organostannique alcoxylé de formule (I) tel que décrit ci-dessus, ledit procédé étant caractérisé en ce qu'il comprend au moins les étapes suivantes :
1) une première étape de mise en réaction, dans un solvant organique, d'un support solide de formule (II) suivante :

   Sup-Z-R⁴ (II)

   dans laquelle :
   - Sup est un support solide macromoléculaire tel que défini ci-dessus pour les réactifs de formule (I), ledit support étant insoluble dans ledit solvant organique ;
   - Z a la même signification que celle indiquée ci-dessus pour les réactifs de formule (I),
   - R⁴ représente un atome d'halogène tel que le chlore, le brome, ou un groupement sulfonate choisi parmi les groupements mésylate, triflate et alylsulfonate ;
   avec un composé organostannique de formule (III) suivante :

   R¹R²SnArM (III)

   dans laquelle :
   - R¹ et R² ont la même signification que celle indiquée ci-dessus pour le réactif de formule (I),
   - Ar est un groupement phényle substitué ou non,
   - M est un métal choisi parmi le lithium, le sodium, le potassium, le magnésium et le cuivre
   pour obtenir un composé organostannique supporté de formule (IV) suivante : 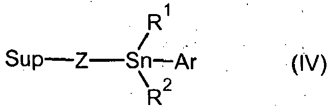 dans laquelle Sup, Z, R¹, R² et Ar ont la même signification que celle indiquée ci-dessus pour les composés de formules (II) et (III) ;
2) une deuxième étape, consistant à faire réagir le composé de formule (IV) obtenu ci-dessus à l'issue de la première étape, dans un solvant organique, avec un composé de formule X₂ choisi parmi I₂, ICI, Cl₂ et Br₂ ou avec un acide de Bronsted de formule HX dans laquelle X = Cl⁻, Br⁻, CF₃COO⁻, ArSO₃⁻, ou RCOO⁻ avec R = alkyle ou aryle, pour obtenir un composé de formule (V) suivante : 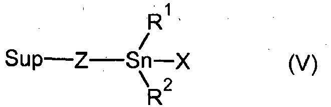 dans laquelle Sup, Z, R¹ et R² ont la même signification que celle indiquée ci-dessus pour les composés de formules (II) et (III) et X est tel que défini ci-dessus ; et
3) une troisième étape, consistant à introduire le composé de formule (V) obtenu ci-dessus à la deuxième étape dans un solvant hydroalcoolique en présence d'une base forte, à soumettre le mélange résultant à une agitation pendant une durée de 6 à 24 heures ; puis à ajouter un carbonate de formule (VI) suivante : 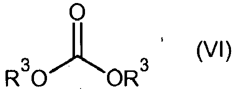
dans laquelle les radicaux R³ sont identiques et ont la même signification que celle indiquée ci-dessus pour les réactifs de formule (I), pour obtenir le réactif de formule (I) attendu.

Les supports solides de formule (II) utilisables dans la première étape peuvent être facilement obtenus par fonctionnalisation d'un support polymère Sup avec un bras de liaison Z, puis avec la partie stannique selon des méthodes classiques et bien connues de l'homme du métier.

Le support polymère Sup se présente généralement sous la forme de particules de taille variable.

A titre de support solide de formule (II), on peut notamment citer les supports de polystyrène réticulé, par exemple par du divinylbenzène. Un tel support correspond par exemple au produit connu sous la dénomination commerciale Amberlite ® XE 305 par la société Rohm and Haas ou au produit PL-PS/DVB MP Resin commercialisé par Polymer Laboratories. Ces produits commerciaux sont ensuite fonctionnalisés par exemple par des groupements chlorobutyle (pour obtenir un bras de liaison de type Z-1 avec n = 4) selon des méthodes déjà décrites dans la littérature.

Les composés organostanniques de formule (III) sont des produits connus. Leur procédé de préparation est par exemple décrit à partir d'un composé de formule R¹R²SnArX et de lithium ou bien à partir d'un composé de formule R¹R²SnArH et de diisopropylamidure de lithium dans le tetrahydrofurane (THF) ou bien encore à partir d'un composé de formule R¹R²SnArH et d'hydrure de sodium ou de potassium dans le THF ou le diméthoxyéthane (DME).

Le solvant utilisé lors de la première étape est de préférence choisi parmi le tetrahydrofurane (THF), le 2-méthyl-tétrahydrofurane, le diméthoxyéthane (DME), et le dioxane. Parmi ces solvants, le THF est particulièrement préféré.

La première étape est de préférence réalisée à une température croissant progressivement de 0°C à 25°C sur une durée de 16 à 24 heures.

Le solvant organique utilisé lors de la deuxième étape est de préférence choisi parmi les alcools inférieurs tels que l'éthanol, l'isopropanol ou le propanol. L'éthanol est particulièrement préféré.

La deuxième étape est de préférence réalisée à une température variant de 40 à 80 °C pendant une durée de 12 à 24 heures.

Le solvant hydroalcoolique utilisé lors de la troisième étape est de préférence choisi parmi les mélanges d'eau et d'au moins un alcool inférieur tel que par exemple l'éthanol.

La troisième étape est de préférence réalisée à une température comprise entre 80 et 140°C (selon la nature de R³) pendant une durée de 6 à 24 heures.

Lorsque la synthèse est terminée, le réactif de formule (I) ainsi obtenu peut être facilement séparé du milieu réactionnel dans lequel il a été synthétisé et dans lequel il est insoluble, par exemple par filtration, décantation ou sédimentation, etc...

Les composés organostamiques alcoxylés de formule (I) peuvent avantageusement être utilisés pour catalyser la synthèse de composés organiques en phase hétérogène.

La présente invention a donc également pour objet l'utilisation d'un composé organostannique alcoxylé de formule (I) tel que défini précédemment, à titre de catalyseur, dans un procédé de synthèse de composés organiques en phase hétérogène, en particulier pour catalyser la synthèse de tétrazoles 5-substitués ou 1,5-disubstitués.

L'invention a enfin pour objet un procédé de synthèse, en phase hétérogène, de tétrazoles de formules (XIIIa) et (XIIIb) suivantes : 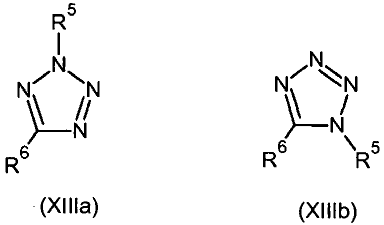 dans lesquelles :
- R⁵ représente un atome d'hydrogène ou un groupement protecteur ;
- R⁶ représente un radical alkyle linéaire en C₁-C₂₄, un radical alkyle ramifié ou cyclique en C₃-C₂₄ ; un groupement aryle, arylalkyle ou hétéroaryle substitué ou non ;
   caractérisé en ce que ledit procédé comprend :
   1) au moins une première étape consistant à faire réagir, dans un solvant organique, sous atmosphère inerte et à une température supérieure ou égale à 110°C, un nitrile de formule (VIII) suivante :

      R⁶-CN (VII)

      dans laquelle R⁶ a la même signification que celle indiquée ci-dessus pour les composés de formule (XIIIa) et (XIIIb), avec un azoture stannique de formule (X) suivante : 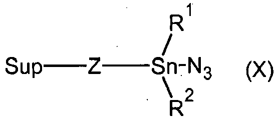 dans laquelle Sup, Z, R¹ et R² ont la même signification que celle indiquée ci-dessus pour les composés de formule (I) ci-dessus,
      ledit réactif de formule (X) étant généré *in situ* par réaction du réactif organostannique alcoxylé correspondant de formule (I) telle que définie précédemment et d'un azoture de trialkylsilyle de formule (IX) suivante : 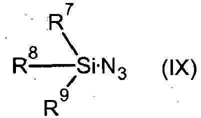 dans laquelle les radicaux R⁷, R⁸ et R⁹, identiques ou différents représentent un radical alkyle linéaire en C₁-C₁₂ (de préférence en C₁-C₄) ou un radical alkyle ramifié ou cyclique en C₃-C₁₂,
      pour obtenir un tétrazole de formule (XIIa) ou (XIIb) suivantes : 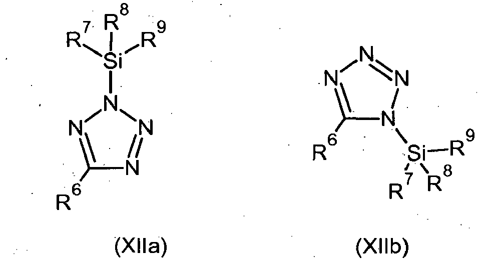 dans lesquelles R⁶ a la même signification que dans la formule (VIII) ci-dessus et R⁷, R⁸ et R⁹ ont la même signification que dans la formule (IX) ci-dessus), après une réaction d'échange des stannyltétrazoles de formules (XIa) ou (XIb) suivantes : 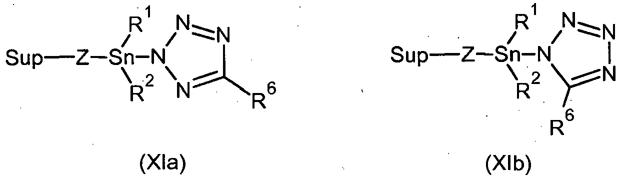 dans lesquelles les radicaux R¹, R² et R⁶ ont les mêmes significations que celles indiquées ci-dessus pour les composés de formules (X) et (VIII) respectivement, avec le composé de formule (IX), et
   2) au moins une deuxième étape consistant :
      i) soit, à hydrolyser le composé de formules (XIIa) ou (XIIb) obtenu ci-dessus à l'étape 1) en milieu acide, pour obtenir un composé de formules (XIIIa) ou (XIIIb) telles que définies précédemment dans lesquelles R⁵ est un atome d'hydrogène, ou
      ii) soit, lorsque l'on souhaite obtenir un composé de formule (XIIIa) ou (XIIIb) dans lequel R⁵ est différent d'un atome d'hydrogène, à faire réagir ledit composé de formule (XIIa) ou (XIIb) obtenu ci-dessus à l'étape 1) avec un halogénure de formule (XIV) suivante :

         X'-R⁵ (XIV)
dans laquelle X' est un atome d'halogène choisi parmi le chlore et le brome et l'iode et R⁵ a la même signification que celle indiquée ci-dessus pour les composés de formules (XIIIa) et (XIIIb),
pour obtenir un composé de formule (XIIIa) ou (XIIIb) dans lesquelles R⁵ a la même signification que dans la formule (XIV) ci-dessus.

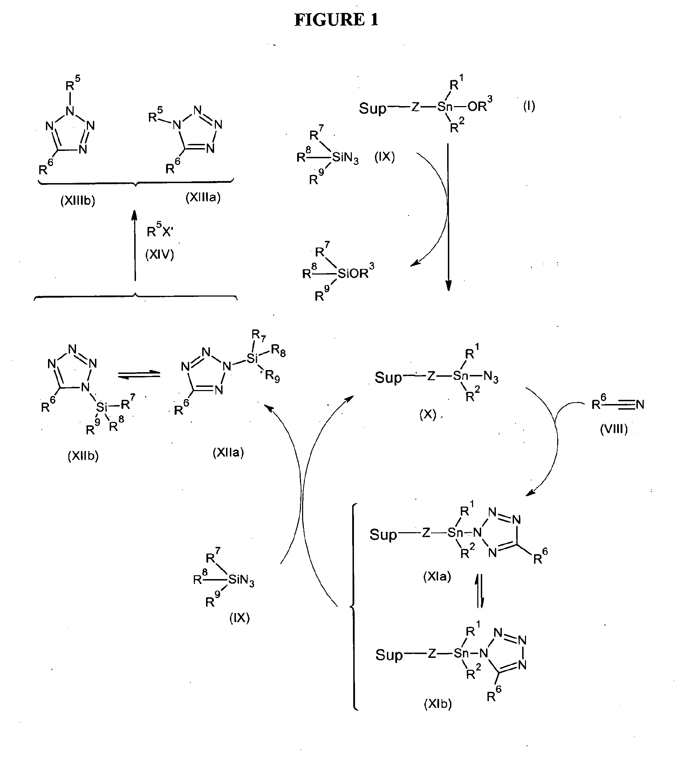

Ce procédé peut être représenté par le schéma réactionnel 1 figurant sur la figure 1 annexée et selon lequel on voit que le composé organostannique de formule (I) réagit avec un azoture de trialkyle silyle de formule (IX) pour donner l'azoture stannique réactif de formule (X) qui va ensuite réagir avec le nitrile de formule (VIII) selon une réaction de cycloaddition pour donner un intermédiaire de formule (XI) dans laquelle le radical R⁶ a la même signification que dans la formule (VIII) et les radicaux R¹ et R² ont la même signification que dans la formule (I). Le composé de formule (XI), en présence d'une deuxième molécule d'azoture de trialkylsilyle de formule (IX), permet d'une part de régénérer l'espèce réactive de formule (X) et de libérer le produit de la réaction sous forme d'un tétrazole silylé de formules (XIIa) ou (XIIb) qui conduit ensuite, dans une dernière étape à un 1*H*-tétrazole 5-substitué de formule (XIIIa) ou (XIIIb) dans lequel R⁵ est un atome d'hydrogène lorsque l'on réalise une protodésilylation, ou bien à un tétrazole 1,5-disubstitué de formule (XIIIa) ou (XIIIb) lorsque R⁵ est différent d'un atome d'hydrogène et que l'on fait réagir l'intermédiaire de formule (XIIa) ou (XIIb) avec un halogénure de formule (XIV).

Le procédé conforme à l'invention permet d'accéder à des molécules comportant une fonction tétrazole de façon efficace et économique puisqu'il utilise de façon transitoire un azidure organostannique supporté, ce qui permet de s'affranchir des inconvénients liés à la purification des produits finaux tout en minimisant leur contamination par des résidus organostanniques. En effet, à l'issue de la réaction, les composés de formules (XIIIa) ou (XIIIb) et le composé stannique de formule (X) sont facilement séparables, par exemple par simple filtration. Les rendements obtenus peuvent être très élevés (de l'ordre de 80 à 90 % environ pour les composés où R⁶ = aryle) bien que la réaction fasse intervenir un réactif immobilisé sur un support solide et qu'elle soit réalisée en phase hétérogène. De plus, le réactif de formule (I) peut-être utilisé en faible quantité puisque (X) intervient dans la réaction en tant que catalyseur, ce qui associé à sa nature supportée permet d'éradiquer de façon très efficace la concentration de résidus organostanniques dans les tétrazoles synthétisés.

La nature du groupement protecteur mentionné pour R⁵ n'est pas critique. Il peut être choisi parmi les groupements protecteurs connus de l'homme du métier et tels que décrits par T P. G. M. Wuts, T. W. Greene, dans « Greene's Protective Groups in Organic Synthesis », Wiley-Interscience, New York, 4th edition, 2006**.** Parmi ces groupements protecteurs, on peut notamment mentionner les radicaux alkyle ramifiés en C₃-C₄ tel que *tert*-butyle ; les radicaux alkyle en C₁ ou C₂ qui sont mono, di ou trisubstitués par des phényle, benzyle dans lesquels l'aryle est non substitué ou substitué par un ou plusieurs groupements choisi parmi les radicaux alkyle en C₁-C₁₂, hydroxyle, alcoxy en C₁-C₁₂, alcanoyloxy en C₁-C₁₂, halogène, nitro, cyano et trifluorométhyle ; picolinyle, pipéronyle ; cumyle ; allyle ; cinnamoyle ; fluorényle ; silyle tel que trialkyle silyle en C₁-C₄ comme par exemple triméthylsilyle, triéthylesilyle et *tert*-butyldiméthylsilyle ou di-C₁-C₄-alkylphénylesilyle comme par exemple diméthylphénylsilyle ; alkylsulfonyle en C₁-C₁₂ ; arylsulfonyle dans lequel le cycle phényle est non substitué ou substitué par un ou plusieurs groupements choisis parmi les radicaux alkyle en C₁-C₁₂, hydroxyle, alcoxy en C₁-C₁₂, alcanoyloxy en C₁-C₁₂, halogène, nitro, cyano, fluoroalkyle, trifluorométhyle ; alcanoyle tel que acétyle, valéroyle ; alcanoyle en C₁-C₇ comme par exemple méthoxyle, éthoxyle ou *tert*-butoxycarbonyle ou allyloxycarbonyle.

Les groupements protecteurs privilégiés sont *tert*-butyle, benzyle, *p*-méthoxybenzyle, 2-phénylpropyl, diphénylméthyle, di-(*p*-méthoxyphényl)méthyle, trityle, *p*-méthoxyphényle, diphénylméthyle, diphényl-(4-pyridyl)méthyle, benzyloxyméthyle, méthoxyméthyle, éthoxyméthyle, méthylthiométhyle, 2-tétrahydropyranyle, allyle, triméthylsilyle et triéthylsilyle.

Les radicaux alkyle et alcoxy mentionnés à titre de substituant pour le radical R⁶ ont de préférence de 1 à 12 atomes de carbone. Parmi de tels groupements, on peut en particulier mentionner les groupements méthyle, méthoxy, éthyle, éthyloxy, propyle, propyloxy, butyle et butyloxy. Les radicaux alkyle linéaires, ramifiés et cycliques mentionnés pour R⁶ peuvent être eux-mêmes éventuellement substitués par un ou plusieurs radicaux choisis parmi un atome d'halogène (chlore, brome, fluor et éventuellement l'iode) et les radicaux hydroxyle, nitro, carboxyle alkyle, alcoxyle, aryle, alcényle, alcynyle, alcoxycarbonyle, trifluorométhyle, fluoroalkyle , aminocarbonyle, sulfonyle et cyano.

A titre de radical aryle mentionné pour R⁶, on peut en particulier citer les groupements naphtylé, anthranyle, phénantryle, o-tolyle, p-tolyle, xylyle, éthylphényle, mésityle, phényle, biphényle, benzoyle dans lesquels les noyaux aromatiques naphtyle, anthranyle, phénantryle, phényle et benzyle peuvent être substitués par un ou plusieurs atomes d'halogène, et/ou par un ou plusieurs groupements choisis parmi les radicaux hydroxyle, nitro, carboxyle, carbonyle, alkyle, fluoroalkyle, trifluorométhyle, alcoxy, alcoxycarbonyle, amido, amino, nitro, cyano, carboxy, aryle, alcényle, alcynyle.

A titre de radical hétéroaryle mentionné pour R⁶, on peut en particulier citer les groupements pyridyle, thiényle, furyle, bipyridyle, terpyridyle qui peuvent être éventuellement substitués par un ou plusieurs atomes d'halogène, et/ou par un ou plusieurs groupements choisis parmi les radicaux hydroxyle, nitro, carboxyle, carbonyle, alkyle, fluoroalkyle, trifluorométhyle, alcoxy, silyloxy, alcoxycarbonyle, amido, amino, nitro, cyano, carboxy, aryle, alcényle et alcynyle.

Parmi les atomes d'halogène mentionnés à titre de substituant du radical R⁶ des composés de formule (VIII), on peut en particulier citer le chlore, le fluor et le brome, le chlore étant particulièrement préféré.

Lorsque R⁵ est un atome d'hydrogène, les sels des composés de formule (XIIIa) ou (XIIIb) sont obtenus en faisant réagir le composé de formule (XIIIa) ou (XIIIb) avec une base. Parmi les sels d'addition obtenus avec une base, on peut en particulier mentionner les sels de métaux alcalins, les sels d'ammoniums et les sels d'amines organiques.

Selon une forme de réalisation préférée du procédé conforme à la présente invention, les radicaux alkyle mentionnés pour les substituants R⁷, R⁸ et R⁹ des composés de formule (IX) sont choisis parmi les radicaux méthyle, éthyle, propyle, butyle, le radical méthyle étant particulièrement préféré.

Parmi les composés de formule (IX) ci-dessus, on préfère ceux dans lesquels les radicaux R⁷, R⁸ et R⁹ sont identiques. Un composé de formule (IX) particulièrement préféré selon l'invention est le composé dans lequel R⁷ = R⁸ = R⁹ = méthyle (Me).

Parmi les composés de formule (X), ceux dans lesquels R¹ = R² = méthyle ou éthyle sont préférés.

Selon une forme de réalisation préférée du procédé conforme à l'invention, le composé de formule (I) est utilisé en quantité catalytique. Au sens de la présente invention, par qualité catalytique, on entend une quantité de catalyseur variant de préférence de 0,05% mol à 15% molaire.

L'atmosphère inerte dans laquelle est réalisé le procédé conforme à l'invention est de préférence une atmosphère d'argon ou de diazote.

Selon une forme de réalisation préférée de l'invention, la première étape du procédé de préparation des composés de formule (XIIIa) ou (XIIIb) est réalisée à une température pouvant varier de 130 à 140°C.

Le solvant organique est de préférence choisi parmi le diméthylformamide, le dibutyléther, le diglyme, le triglyme et les xylènes.

La durée de la première étape peut varier de 30 min à 20 heures ; elle est généralement de l'ordre de 4 heures environ.

Lors de la deuxième étape, l'hydrolyse en milieu acide des composés de formules (XIIIa) ou (XIIIb) est de préférence réalisée par ajout, au milieu réactionnel, d'un acide organique ou inorganique, de préférence choisi parmi l'acide chlorhydrique, l'acide sulfurique et l'acide trifluoroacétique.

A l'issue de la synthèse, le composé de formules (XIIIa) ou (XIIIb) obtenu, en phase liquide, peut être facilement séparé du composé organostannique de formule (X) présent sous forme solide dans le milieu réactionnel, par toute technique appropriée connue de l'homme du métier, telle que par exemple par filtration, décantation ou sédimentation. Le composé de formule (I) peut alors ensuite être régénéré par exemple par traitement avec une base forte telle que la soude (en particulier NaOH 4M dans un mélange éthanol/eau 1 :1) puis réaction avec le réactif de formule (VI).

Le composé de formules (XIIIa) ou (XIIIb) attendu est ensuite récupéré après traitement basique du milieu réactionnel, puis traitement acide et cristallisation en phase aqueuse.

Un autre objet de l'invention est l'utilisation d'un composé organostannique alcoxylé de formule (I) tel que défini précédemment à titre de catalyseur pour la synthèse, en phase hétérogène, d'un tétrazole choisi parmi le 5-phényl-1*H-*tétrazole, le 5-(4-méthylphényl)-1*H*-tétrazole, le 5-(4-méthoxyphényl)-1*H-*tétrazole, le 5-(3-méthoxyphényl)-1*H*-tétrazole, le 5-(4-nitrophényl)-1*H*-tétrazole, l'acide 4-(1*H*-tetrazol-5-yl)benzoïque, le 5-(3-chlorophényl)-1*H*-tétrazole, le 5-(4-chlorophényl)-1*H*-tétrazole, le 5-(4-hydroxyphényl)-1*H*-tétrazole, le 5-(3-trifluorométhylphényl)-1*H*-tétrazole, le 5-naphtyl-1*H*-tétrazole, le 5-butyl-1*H-*tétrazole, le 5-octyl-1*H*-tétrazole, le 5-(2-bromophényl)-1*H*-tétrazole et le 5-biphényl-2-yl-1*H*-tétrazole.

La présente invention est illustrée par les exemples de réalisation suivants, auxquels elle n'est cependant pas limitée.

### EXEMPLES

Matières premières utilisées dans les exemples qui suivent :
- Produits chimiques : diméthyldichloroétain (CAS : 753-73-1), bromure de phénylmagnésium (3,0 M dans l'éther diéthylique, diéthyl éther, CAS : 100-58-3), hydrure de lithium et d'aluminium (CAS : 16853-85-3), diisopropylamine (CAS : 108-18-9), butyllithium (CAS : 109-72-8), tetrahydrofurane (CAS : 109-99-9), éthanol (CAS: 64-17-5), méthanol (CAS : 67-56-1), acide chlorhydrique (CAS : 7647-01-0), chlorure de sodium (7647-14-5), diiode (CAS : 7553-56-2), thiosulfate de sodium (CAS : 7772-98-7), diéthyléther (60-29-7), le sulfate de sodium (7487-88-9), hydroxyde de sodium (CAS : 1310-73-2), diméthylcarbonate (CAS : 616-38-6 ), ces produits chimiques étant tous commercialisés par la société Aldrich.
- Billes de polystyrène réticulé, vendues sous la dénomination commerciale Amberlite ® XE 305 par la société Rohm and Haas ou sous la dénomination commerciale PL-PS/DVB MP Resin par la société Polymer Laboratories ;

### Exemples 1 à 12 : Synthèse de 1H-tétrazoles substitués en position 5 par un groupement aromatique

Dans cet exemple, on a préparé des 1*H*-tétrazoles substitués en position 5 par un groupement phényle substitué ou non, selon le schéma réactionnel général suivant : 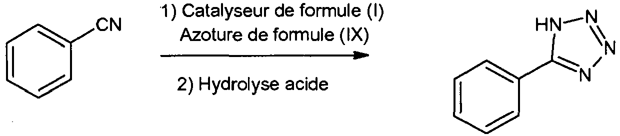 consistant à mettre en réaction un catalyseur de formule (I) (quantité catalytique) avec un azoture de trialkyle silyle de formule (IX) et le nitrile de formule (VIII) correspondant au tétrazole de formule (XIII) que l'on souhaite obtenir, sous atmosphère inerte à reflux dans le solvant organique (éther n-butylique (Bu₂O)) pendant environ 16 heures.

*In situ,* le catalyseur de formule (I) réagit avec l'azoture de trialkyle silyle de formule (IX) pour donner l'espèce réactive de formule (X) correspondante (azoture d'étain supporté) qui va réagir avec le nitrile de formule (VIII) utilisé selon une réaction de cycloaddition pour donner l'intermédiaire de formule (XI) correspondant. Ce dernier, en présence d'une deuxième molécule d'azoture de trialkyle silyle de formule (IX), permet d'une part de régénérer l'espèce réactive de formule (X) et d'autre part de libérer le produit de la réaction sous forme d'un tétrazole silylé de formule (XII). Après un traitement acide puis un traitement basique, le tétrazole final de formule (XIII) attendu est aisément obtenu par cristallisation en phase aqueuse et filtration.

Le catalyseur supporté utilisé dans ces exemples est un catalyseur de formule (I) avec pour bras de liaison Z-1 dans lequel n = 4 et R¹ = R² = R³ = CH₃, et dans lequel le support solide insoluble est du polystyrène réticulé par du divinylbenzène.

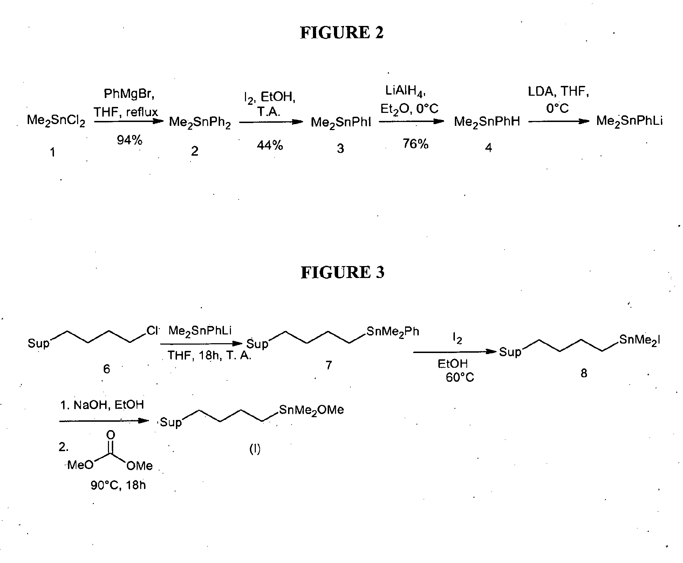

Il a été synthétisé selon les schémas réactionnels 2 et 3 reportés respectivement sur les figures 2 et 3 annexées :

### 1) Préparation du diméthyldiphénylétain 2

80,5 mmol de diméthyldichloroétain **1** (Me₂SnCl₂) dans 120 mL tetrahydrofurane anhydre (THF) ont été ajoutés au goutte à goutte et sous argon à 70 mL d'une solution de bromure de phénylmagnésium (PhMgBr) à 2,3 M (161 mol, 2 équivalents) dans l'éthanol. Le mélange réactionnel a été chauffé à 80°C pendant 3 heures et la réaction a été suivie par chromatographie sur couche mince (CCM) afin de suivre la disparition complète des réactifs de départ. Le milieu réactionnel a ensuite été hydrolysé avec de l'acide chlorhydrique (HCl) 1M (80 mL) et la phase aqueuse a été extraite avec du diéthyléther (3 x 150 mL). Les phases organiques ont été lavées avec du NaCl (400 mL), séchées sur MgSO₄ et concentrées sous vide. Le résidu a été purifié par chromatographie sur gel de silice et le composé **2** attendu a été obtenu sous la forme d'une huile incolore avec un rendement de 94%.

RMN ¹H (300 MHz, CDCl₃) δ = 0,51 (6H, s, *²J*^{*117*/}*¹¹⁹_{Sn-H}* = 56,6/58,8) ; 7,3-7,6 (10H, m) ppm.

RMN ¹³C (300 MHz, CDCl₃) δ = -9,7 (*¹J*_{*Sn*-*C*} = 363) ; 128,6 ; 128,9 ; 136,6 (*²J*_{*Sn-*C}= 36) ; 141 ppm.

RMN ¹¹⁹Sn (300 MHz, CDCl₃) δ = -58,6 ppm.

### 2) Préparation l'iodure de diméthylphénylétain 3

A une solution de 12,4 mol de diméthyldiphénylétain **2** dans le méthanol (0,3 M, 21 mL), on a ajouté, dans l'obscurité, une solution d'iode (3,19 g, 12,6 mmol, 1,02 eq.) dans le méthanol (0,3M, 21 mL), sous argon et à température ambiante. Le mélange réactionnel a été mélangé pendant 18 heures à température ambiante et partiellement concentré sous vide. Le mélange résultant a ensuite été dilué avec une solution aqueuse saturée de Na₂S₂O₃ et la phase aqueuse a été extraite avec du diéthyléther (3 x 200 mL). Les phases organiques ont ensuite été lavées avec du NaCl (300 mL), séchées sur MgSO₄ et concentrées sous vide. L'iodobenzène restant dans le résidu résultant a été distillé (point d'ébullition à 0,68 mbar : 21,9°C) et le composé **3** attendu a été obtenu sans autre purification supplémentaire sous la forme d'une huile jaune avec un rendement de 44 %.

RMN ¹H (300 MHz, CDCl₃) δ = 1,05 (6H, s, ²*J* ^{117/119}_{SnH} = 55.4/58), 7,34-7,5 (3H, m), 7,6 (2H, d, ³*J* = 7,6) ppm.

RMN ¹¹⁹Sn (300 MHz, CDCl₃) δ = -17 ppm.

### 3) Préparation de l'hydrure de diméthylphénylétain 4 (Me₂SnPhH)

A une suspension de 44,1 mmol d'hydrure d'aluminium et de lithium (LiAlH₄) (2,5 éq.) dans 75 mL d'éthanol, ont été ajoutées au goutte à goutte, à 0°C sous argon, 17,6 mmol de composé **3** obtenu ci-dessus à l'étape précédente. Le mélange réactionnel a été mélangé pendant 2 heures à 0°C dans l'obscurité puis hydrolysé avec 15 mL d'eau. La phase aqueuse a été extraite avec du diéthyléther (3 x 50 mL) et les phases organiques ont été lavées avec une solution saturée de NaCl (40 mL), séchées sur MgSO₄ et concentrées sous vide. Le composé **4** a été obtenu sous forme d'une huile incolore avec un rendement de 76 %.

RMN ¹H (300 MHz, C₆D₆) δ = 0,39 (6H, s, *²J_{Sn-H}* = 56), 5,66 (1H, s, ¹*J* ^{117/119}_{SnH} = 1725/1810, Sn*H*), 7,39 (3H, m), 7,62 (2H, d, ³*J*= 7,3) ppm.

RMN ¹³C (300 MHz, C₆D₆) δ = -11 (²*J _{Sn-C}* = 351) ; 129,3 ; 137,3 (*²J_{Sn-C}*= 38,5), 137,8 ; 140 ppm.

RMN ¹¹⁹Sn (300 MHz, C₆D₆) δ = -121 ppm.

### 4) Préparation du poly [4-(diméthylphénylstannyl)butyl]styrène 7

A une solution de 30,5 mmol de diisopropylamidure de lithium (LDA) dans le THF anhydre, ont été ajoutées lentement, à 0°C et sous argon, 27,7 mmol du composé **4** obtenu ci-dessus à l'étape précédente. Le mélange résultant **5** a été mélangé pendant 1 heure à 0°C, puis on y a ajouté 7,4 g de polymère **6** à l'état sec. On a laissé le mélange revenir à température ambiante, puis celui-ci a été maintenu sous agitation pendant 18 heures. Le polymère résultant a été successivement lavé avec 60 mL d'un mélange de THF et d'eau (1:1 ; v:v), 6 x 60 mL de THF, 4 x 60 mL d'éthanol absolu avant d'être séché sous vide (0,5 mbar) à 60°C pendant 5 heures. Le polymère **7** a été obtenu sous la forme d'une résine blanche (9,5 g).

RMN MAS ¹¹⁹Sn δ = -33 ppm.

### 5) Préparation du poly [4-(iododiméthylstannyl)butyl]styrène 8

On a ajouté à une solution d'iode (3,81 g, 15,0 mmol) dans 50 mL d'éthanol absolu, 10,0 g du polymère **7** obtenu ci-dessus à l'étape précédente. Le mélange résultant a été maintenu sous agitation à 60°C pendant 18 heures dans l'obscurité. Le polymère a ensuite été lavé successivement avec 60 mL d'un mélange THF/solution aqueuse saturée de Na₂S₂O₃ (1:1 ; v:v), 6 x 60 mL de THF, 4 x 60 mL d'éthanol absolu avant d'être séché sous vide (0,5 mbar) à 60°C pendant 5 heures. Le polymère **8** a été obtenu sous forme d'une résine jaune pâle.

RMN MAS ¹¹⁹Sn δ = + 49 ppm.

### 6) Préparation du composé 9 (réactif de formule (I))

On a ajouté à une solution de 2 g (2,36 mmol) de polymère **8** obtenu ci-dessus à l'étape précédente dans 10 mL d'éthanol absolu, une solution de soude 4 M (2,36 mL, 9,5 mmol, 4 éq.) dans un mélange éthanol/eau (1:1 ; v:v). Le mélange réactionnel a été maintenu sous agitation à température ambiante pendant 36 heures. Le matériau insoluble a été successivement lavé avec 4 x 30 mL d'eau, puis 7 x 30 mL d'éthanol avant d'être séché sous vide (0,5 mbar) à 60°C pendant 5 heures. Le polymère résultant a ensuite été introduit dans un ballon à fond rond, dans lequel ont été ensuite ajoutés 2 mL de diméthylcarbonate. Le mélange réactionnel a été maintenu sous agitation pendant 18 heures à 90°C. Après filtration, le réactif de formule (**I**) (composé **9**) attendu a été lavé avec 70 mL de THF, puis avec 70 mL d'éthanol avant d'être séché sous vide (0,5 mbar) à 60°C pendant 5 heures.

RMN MAS ¹¹⁹Sn δ = + 110 ppm.

La synthèse des différents tétrazoles a ensuite été réalisée de la façon suivante :
Dans un bicol équipé d'un réfrigérant, on a introduit le nitrile de formule (VIII) (1,0 mmol) correspondant au tétrazole de formule (XIII) attendu, le réactif de formule (I) tel que préparé comme indiqué ci-dessus (100 mg, 0,1 mmol) ainsi que du (CH₃)₃SiN₃ (azoture de trimétliylsilyle (IX)) (0,2 mL, 1,5 mmol) dans 2,5 mL de Bu₂O sous atmosphère inerte. Le milieu réactionnel a été chauffé à reflux sous atmosphère inerte pendant 16 heures et a ensuite été dilué dans l'éther de pétrole (10 mL) et traité par une solution de NaOH 1M (10 mL). Le polymère résiduel a ensuite été filtré et lavé successivement avec NaOH 1M (5 mL), du THF (5 mL) puis de l'éther de pétrole (5 mL). Le filtrat a été récupéré et la phase aqueuse a été séparée puis acidifiée avec HCl à pH 1 à 0°C afin de cristalliser le tétrazole de formule (XIII) attendu. Les cristaux ont ensuite été filtrés et séchés sous vide.

Le tableau 1 ci-après présente les différents tétrazoles de formule (I) synthétisés, ainsi que le rendement de la réaction :

**TABLEAU 1**

| **Exemple** | **Tétrazole de formule (VII)** | | **Rendement en produit isolé (%)** |
|---|---|---|---|
| **1** | 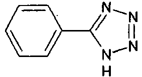 | 5-phényl-1*H*-tétrazole | 95 |
| **2** | 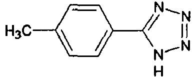 | 5-(4-méthylphényl)-1*H*-tétrazole | 91 |
| **3** | 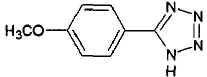 | 5-(4-méthoxyphényl)-1*H*-tétrazole | 89 |
| **4** | 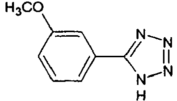 | 5-(3-méthoxyphényl)-1*H*-tétrazole | 78 |
| **5** | 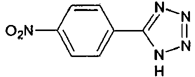 | 5-(4-nitrophényl)-1*H*-tétrazole | 90 |
| **6** | 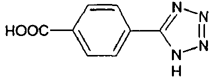 | Acide 4-(1*H*-tetrazol-5-yl)benzoïque | 81 |
| **7** | 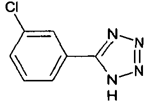 | 5-(3-chlorophényl)-1*H*-tétrazole | 83 |
| **8** | 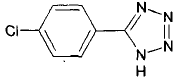 | 5-(4-chlorophényl)-1*H*-tétrazole | 86 |
| **9** | 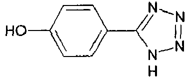 | 5-(4-hydroxyphényl)-1*H*-tétrazole | 82 |
| **10** | 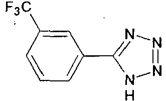 | 5-(3-trifluorométhylphényl)-1*H*-tétrazole | 87 |
| **11** | 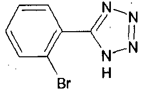 | 5-(2-bromophényl)-1*H*-tétrazole | 72 |
| **12** | 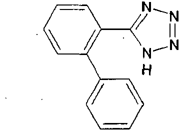 | 5-biphényl-2-yl-1*H*-tétrazole | 63 |

### Caractérisation des composés

### 5-phényl-1H-tétrazole (Ex. 1)

Solide blanc, Température de fusion : 210-211 °C;

RMN ¹H (300 MHz, d₆-DMSO) : δ = 8,02-7,98 (m, 2H), 7,58-7,54 (m, 3H), ppm ;

RMN ¹³C (75 MHz, d₆-DMSO) : δ = 155,8; 131,6; 129,8; 127,3 ; 124,7 ppm.

### 5-(4-méthylphényl)-1H-tétrazole (Ex. 2)

Solide blanc ; Température de fusion. = 246-248°C

IR (KBr) = 3100-2200 (br), 1614, 1569, 1504, 1163, 1055, 1028, 823, 744 cm-1

RMN ¹H (300 MHz, d₆-DMSO) : 7,92 ppm (d, *J =* 8,0 Hz, 2 H); 7,41 (d, *J*= 8,0 Hz, 2H) ; 2,39 (s, 3 H) ppm.

RMN ¹³C (100 MHz, d₆-DMSO) : 155,0 ; 141,0 ; 129,8 ; 126,8 ; 121,2 ; 21,0 ppm.

### 5-(4-méthoxyphényl)-1H-tétrazole (Ex. 3)

Solide blanc ; Température de fusion : 231-233°C

IR (KBr) : 3200-2300 (br), 1298, 1184, 1035, 750 cm-1

RMN ¹H (300 MHz, d₆-DMSO) : δ = 7,96 (d, *J* = 9,0 Hz, 2H), 7,15 (d, *J=* 9.0 Hz, 2H), 3,85 (s, 3H) ppm ;

RMN ¹³C (100 MHz, d₆-DMSO) : 161,3; 154,6; 128,5; 116,2 ; 114,7 ; 55,4 ppm

### 5-(3-méthoxyphényl)-1H-tétrazole (Ex. 4)

### Solide blanc

RMN ¹H (300 MHz, d₆-DMSO) : δ = 7,64-7,50 (m, 2H), 7,53 (t, J = 7,5 Hz, 1H), 7,17 (ddd, J = 1,5 Hz, J = 3 Hz, J = 9Hz, 1H)

RMN ¹H (75 MHz, d₆-DMSO) : δ = 159,7 ; 130,7 ; 125,3 ; 119,1 ; 117,01 ; 112,1 ; 55,4 ppm.

### 5-(4-nitrophényl)-1H-tétrazole (Ex. 5)

Solide blanc, Température de fusion = 218-220°C

IR (KBr) : 3500-2400 (br), 1604, 1531, 1488, 1338, 1311, 993, 867, 854 cm-1;

RMN ¹H (300 MHz, d₆-DMSO) : δ = 8,45 ppm (d, J = 9,0 Hz, 2H) ; 8,30 (d, J = 9,0 Hz, 2H) ;

RMN ¹³C (75 MHz, d₆-DMSO) : δ = 155,6 ; 148,8 ; 130,7 ; 128,3 ; 124,6 ppm.

### Acide 4-(1H-tetrazol-5-yl)benzoïque (Ex. 6)

RMN ¹H (300 MHz, d₆-DMSO) : δ = 13,1 (large s, 1H) ; 8,1 ppm (m, 4H) ; 3,3 (large s, 1 H)

RMN ¹³C (75 MHz, d₆-DMSO) : δ = 166.6 ; 155,3 ; 132,9 ; 130,2 ; 128,2 ; 127,1

### 5-(3-chlorophényl)-1H-tétrazole (Ex. 7)

RMN ¹H (300 MHz, d₆-DMSO) : 8,07-8,02 (m, 1H) ; 7,66-7,64 (m, 1H), 7,7-7,6 (m, 2H)

RMN ¹³C (75 MHz, d₆-DMSO): 154,8; 133,9; 131,4; 130,9; 126,5; 126,4 ; 125,6

### 5-(4-chlorophényl)-1H-tétrazole (Ex. 8)

Solide blanc ; Température de fusion : 252-254 °C ;

RMN ¹H (300 MHz, d₆-DMSO) : δ = 8,07 (d, *J* = 8,8 Hz, 2H) ; 7,70 (d, *J* = 8,8 Hz, 2H);

RMN ¹³C (75 MHz, d₆-DMSO) : δ = 155,3 ; 136,3 ; 129,9 ; 129,0 ; 123,6.

### 5-(4-hydroxyphényl)-1H-tétrazole (Ex. 9)

Solide blanc, Température de fusion : 236-238 °C ;

IR (KBr): 3600-3200 (br), 1616, 1515, 1471, 1282, 1247, 1080, 995, 842 cm-1 ;

RMN ¹H (300 MHz, d₆-DMSO) : δ = 10,18 (br s, 1H) ; 7,86 (d, *J*= 8.5 Hz, 2 H) ; 6,95 (d, *J*= 8,5 Hz, 2H) ppm

RMN ¹³C (75 MHz, d₆-DMSO) : δ = 160,0; 154,8 ; 128,7; 116,2; 114,6 ppm.

### 5-(4-trifluorométhylphényl)-1H-tétrazole (Ex 10)

RMN ¹H (300 MHz, d₆-DMSO) : 8,35 (2H, m), 7,9 (1H, m), 7,85 (1H, m)

RMN ¹³C (75 MHz, d₆-DMSO) : 152,9 ; 128,5 (q, *J* = 1,2 Hz) ; 128,5 ; 127,8 (q, *J* = 32 Hz) ; 125,4 (q, *J* = 3,8 Hz) ; 123,0 ; 121,3 (q, *J* = 277 Hz) ; 121,0 (q, *J* =4,0 Hz).

### 5-(2-bromophényl)-1H-tétrazole (Ex. 11)

Solide blanc, température de fusion : 179-180°C ;

RMN ¹H (300 MHz, d⁶-DMSO) : 7,5-7,6 (m, 2H) ; 7,7 (dd, *J* = 7,5 ; *J* = 2, 1H);

RMN ¹³C (100 MHz, d⁶-DMSO) : 121,6 ; 126,3 ; 128,1 ; 131,9 ; 132,5 ; 133,4; 154,5.

### 5-'bi-phényl-2-yl-1H-tétrazole (Ex. 12)

Solide blanc, température de fusion : 142-143°C ;

RMN ¹H (300 MHz, d⁶-DMSO) : 7,1 (m, 2H) ; 7,31 (m, 3H) ; 7,58 (m, 2H) ; 7,69 (m, 2H) ;

RMN ¹³C (100 MHz, d⁶-DMSO) : 123,4 ; 127,4 ; 127,5 ; 127,7 ; 128,2 ; 128,7 ; 130,5 (2C), 131,0 ; 139,2 ; 141,5.

### Exemple 13 : Préparation du 5-naphtyle 1H-tetrazole

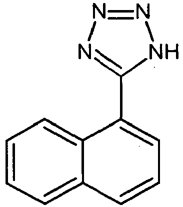

Dans cet exemple, on a préparé le 5-naphtyle-1*H*-tetrazole à partir du nitrile de formule (VIII) correspondant selon le protocole utilisé ci-dessus pour les composés des exemples 1 à 12. On a obtenu le 5-naphtyle-1*H*-tetrazole sous la forme d'un solide blanc avec un rendement de 85 %.

Température de fusion : 205-206°C;

IR (KBr) : 3200-2200 (br), 3060, 1566, 1417, 1249, 1085, 1020, 825, 759 cm⁻¹;

RMN ¹H (300 MHz, d₆-DMSO) δ = 8,57-8,54 (m, 1H), 8,21-8,18 (m, 1H) ; 8,11-8,08 (m,1H) ; 8,00-7,98 (m, 1H) ; 7,74-7,63 (m, 3H) ;

RMN ¹³C (75 MHz, d₆-DMSO) δ = 133,3 ; 131,4 ; 129,9 ; 128,6 ; 128,3 ; 127,6 ; 126,7 ; 125,3 ; 125,1 ; 121,5 ; 114,4.

### Exemple 14 : Préparation du 5-butyl-1H-tétrazole

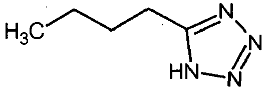

Dans cet exemple, on a préparé le 5-butyl-1*H***-**tetrazole à partir du nitrile de formule (VIII) correspondant selon le protocole utilisé ci-dessus pour les composés des exemples 1 à 12. On a obtenu le 5-butyl-1*H-*tetrazole sous la forme d'un solide blanc avec un rendement de 50 %.

RMN ¹H (300 MHz, d₆-DMSO) : δ = 2,87 (t, J = 7,5 Hz, 2H) ; 1,68 (m, 2H) ; 1,33 (m, 2H) ; 0,9 (t, J = 7.5 Hz, 3H).

RMN ¹³C (75 MHz, d₆-DMSO) : δ =155,9 ; 29,0 ; 22,3 ; 21,4 ; 13,4.

### Exemple 15 : Préparation du 5-octyl-1H-tétrazole

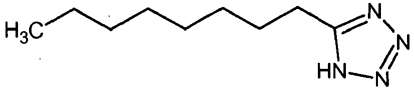

Dans cet exemple, on a préparé le 5-octyl-1*H*-tetrazole à partir du nitrile de formule (VIII) correspondant selon le protocole utilisé ci-dessus pour les composés des exemples 1 à 12. On a obtenu le 5-octyl-1*H*-tetrazole sous la forme d'un solide jaune pâle avec un rendement de 42 %.

RMN ¹H (300 MHz, d₆-DMSO) : 2,85 ; (t, J = 7,5 Hz, 2H) ; 1,68 (m, 2H) ; 1,26 (m, 10H) ; 0,85 (m, 3H).

RMN ¹³C (75 MHz, d₆-DMSO) : 155,9 ; 31,1 ; 28,4 ; 28,3 ; 27,0 ; 22,6 ; 22,0 ; 13,9.

## Revendications

1. Réactif organostannique alcoxylé de formule (I) suivante : 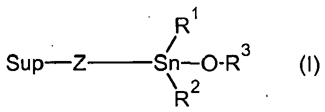 dans laquelle :
- Sup représente un support solide macromoléculaire ;
- Z représente un bras de liaison entre le support solide macromoléculaire Sup et l'atome d'étain, Z étant choisi parmi les groupements de formules (Z-1) à (Z-6) suivantes :
-(CH₂)ₙ- (Z-1) 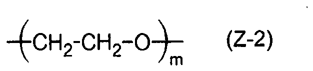 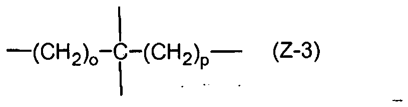 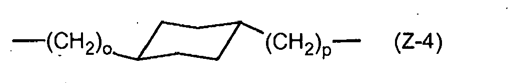 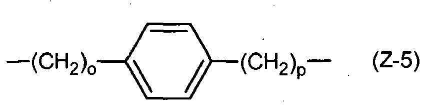
-CH₂-O-(CH₂)_{q}-CH₂-CH₂- (Z-6)
dans lesquelles :
- n et m, indépendamment l'un des l'autre, sont des nombres entiers variant de 2 à 24,
- o et p, indépendamment l'un de l'autre, sont des nombres entiers variant de 1 à 24,
- q est un nombre entier variant de 1 à 24,
- R¹, R² et R³, indépendamment les uns des autres, représentent un radical alkyle linéaire en C₁-C₂₄, un radical alkyle ramifié, ou cyclique en C₃-C₂₄, alcényle linéaire en C₂-C₂₄, alcényle ramifié ou cyclique en C₃-C₂₄, aryle ou arylalkyle.

2. Réactif selon la revendication 1, **caractérisé en ce que** Sup est insoluble dans les solvants organiques.

3. Réactif selon la revendication 1 ou 2, **caractérisé en ce que** le polymère du support solide est choisi parmi les polymères à base de styrène, les poly(phénylène éthers), les poly(phénylène sulfures), et les polyamides.

4. Réactif selon la revendication 3, **caractérisé en ce que** le support polymère est choisi parmi les supports polymères résultant de la copolymérisation du styrène et du divinylbenzène en tant qu'agent réticulant.

5. Réactif selon l'une quelconque des revendications précédentes, **caractérisé en ce qu'**il est choisi parmi les composés dans lesquels :
- Sup est un support solide polymère à base de styrène réticulé ou non ;
- Z = -(CH₂)ₙ avec n = 3 ou 4 ; et
- les radicaux R¹, R², et R³ ont les significations suivantes :
i) R¹ = R² = R³ = méthyle, éthyle, propyle ou butyle ;
ii) R¹ = R² = méthyle et R³ = éthyle, propyle ou butyle ;
iii) R¹ = R² = éthyle et R³ = méthyle, propyle ou butyle ;
iv) R¹ = R² = propyle et R³ = méthyle, éthyle ou butyle ; ou
v) R¹ = R² = butyle et R³ = méthyle, propyle ou éthyle.

6. Procédé de préparation d'un réactif organostannique alcoxylé de formule (I) tel que défini à l'une quelconque des revendications précédentes, **caractérisé en ce qu'**il comprend au moins les étapes suivantes :
1) une première étape de mise en réaction, dans un solvant organique, d'un support solide de formule (II) suivante :
Sup-Z-R⁴ (II)
dans laquelle :
- Sup est un support solide macromoléculaire, ledit support étant insoluble dans ledit solvant organique ;
- Z représente un bras de liaison entre le support solide macromoléculaire Sup et l'atome d'étain, Z étant choisi parmi les groupements de formules (Z-1) à (Z-6) suivantes :
-(CH₂)ₙ- (Z-1) 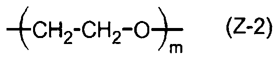 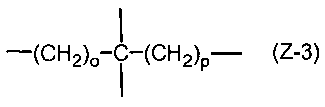 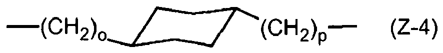 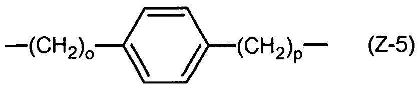
-CH₂-O-(CH₂)_{q}-CH₂-CH₂- (Z-6)
dans lesquelles :
• n et m, indépendamment l'un des l'autre, sont des nombres entiers variant de 2 à 24 ;
• o et p, indépendamment l'un de l'autre, sont des nombres entiers variant de 1 à 24,
• q est un nombre entier variant de 1 à 24,
- R⁴ représente un atome d'halogène ou un groupement sulfonate choisi parmi les groupements mésylate, triflate et arylsulfonate ;
avec un composé organostannique de formule (III) suivante :
R¹R²SnArM (III)
dans laquelle :
- R¹ et R² ont la même signification que celle indiquée à la revendication 1 pour le réactif de formule (I),
- Ar est un groupement phényle substitué ou non,
- M est un métal choisi parmi le lithium, le sodium, le potassium, le magnésium et le cuivre ;
pour obtenir un composé organostannique supporté de formule (IV) suivante : 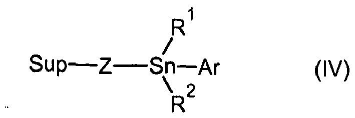 dans laquelle Sup, Z, R¹, R² et Ar ont la même signification que celle indiquée ci-dessus pour les composés de formules (II) et (III) ;
2) une deuxième étape, consistant à faire réagir le composé de formule (IV) obtenu ci-dessus à l'issue de la première étape, dans un solvant organique, avec un composé de formule X₂ choisi parmi I₂, ICI, Cl₂ et Br₂ ou avec un acide de Bronsted de formule HX dans laquelle X = Cl⁻, Br⁻, CF₃COO⁻, ArSO₃⁻, ou RCOO⁻ avec R = alkyle ou aryle, pour obtenir un composé de formule (V) suivante : 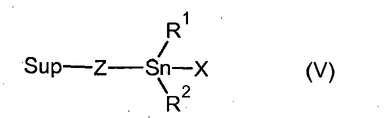 dans laquelle Sup, Z, R¹ et R² ont la même signification que celle indiquée ci-dessus pour les composés de formules (II) et (III) et X est tel que défini ci-dessus ; et
3) une troisième étape, consistant à introduire le composé de formule (V) obtenu ci-dessus à la deuxième étape dans un solvant hydroalcoolique en présence d'une base forte, à soumettre le mélange résultant sous agitation pendant une durée de 6 à 24 heures ; puis à ajouter un carbonate de formule (VI) suivante : 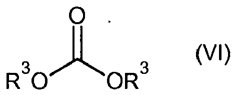 dans laquelle les radicaux R³ sont identiques et ont la même signification que celle indiquée ci-dessus à la revendication 1 pour les réactifs de formule (I), pour obtenir le réactif de formule (I) attendu.

7. Procédé selon la revendication 6, **caractérisé en ce que** le support solide de formule (II) est choisi parmi les supports de polystyrène réticulé par du divinylbenzène, et fonctionnalisés par des groupements chlorobutyle.

8. Procédé selon la revendication 6 ou 7, **caractérisé en ce que** le solvant utilisé lors de la première étape est choisi parmi le tetrahydrofurane, le 2-méthyl- tétrahydrofurane, le diméthoxyéthane, et le dioxane.

9. Procédé selon l'une quelconque des revendications 6 à 8, **caractérisé en ce que** la première étape est réalisée à une température croissant progressivement de 0°C à 25°C sur une durée de 16 à 24 heures.

10. Procédé selon l'une quelconque des revendications 6 à 9, **caractérisé en ce que** la deuxième étape est réalisée à une température variant de 40 à 80 °C pendant une durée de 12 à 24 heures.

11. Procédé selon l'une quelconque des revendications 6 à 10, **caractérisé en ce que** la troisième étape est réalisée à une température comprise entre 80 et 140°C pendant une durée de 6 à 24 heures.

12. Utilisation d'un composé organostannique alcoxylé de formule (I) tel que défini à l'une quelconque des revendications 1 à 5, à titre de catalyseur, dans un procédé de synthèse de composés organiques en phase hétérogène.

13. Utilisation selon la revendication 12, pour catalyser la synthèse de tétrazoles 5-substitués ou 1,5-disubstitués.

14. Procédé de synthèse, en phase hétérogène, de tétrazoles 5-substitués de formules (XIIIa) et (XIIIb) suivantes : 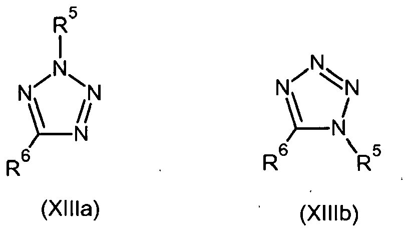 dans lesquelles :
- R⁶ représente un radical alkyle linéaire en C₁-C₂₄, alkyle ramifié ou cyclique en C₃-C₂₄, aryle, arylalkyle ou hétéroaryle substitués ou non ;
- R⁵ représente un atome d'liydrogène ou un groupement protecteur, **caractérisé en ce que** ledit procédé comprend :
1) au moins une première étape consistant à faire réagir, dans un solvant organique, sous atmosphère inerte et à une température supérieure ou égale à 110°C un nitrile de fonnule (VIII) suivante :
R⁶-CN (VIII)
dans laquelle R⁶ a la même signification que celle indiquée ci-dessus pour les composés de formule (XIIIa) et (XIIIb), avec un azoture stannique de formule (X) suivante : 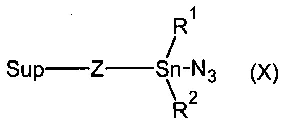 dans laquelle Sup, Z, R¹ et R² ont la même signification que celle indiquée à la revendication 1 pour les composés de formule (I) ci-dessus,
ledit réactif de formule (X) étant généré *in situ* par réaction du réactif organostannique alcoxylé correspondant de formule (I) telle que définie à la revendication 1 et d'un azoture de trialkylsilyle de formule (IX) suivante : 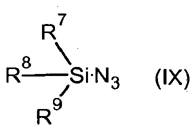 dans laquelle les radicaux R⁷, R⁸ et R⁹, identiques ou différents représentent un radical alkyle linéaire en C₁-C₁₂ (de préférence en C₁-C₄) ou un radical alkyle ramifié ou cyclique en C₃-C₁₂,
pour obtenir un tétrazole de formule (XIIa) ou (XIIb) suivantes : 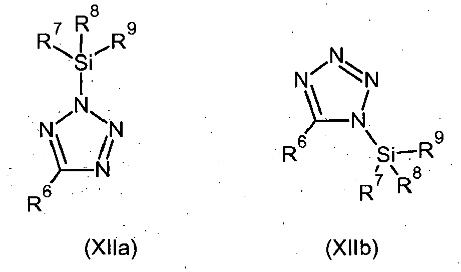 dans lesquelles R⁶ a la même signification que dans la formule (VIII) ci-dessus et R⁷, R⁸ et R⁹ ont la même signification que dans la formule (IX) ci-dessus), après une réaction d'échange des stannyltétrazoles de formules (XIa) ou (XIb) suivantes : 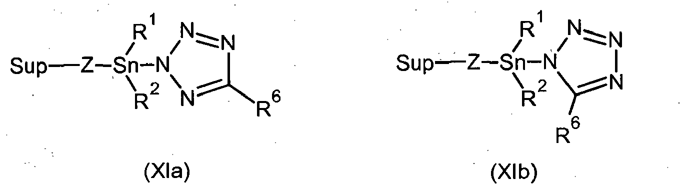 dans lesquelles les radicaux R¹, R² et R⁶ ont les mêmes significations que celles indiquées ci-dessus pour les composés de formules (X) et (VIII) respectivement, avec le composé de formule (IX), et
2) au moins une deuxième étape consistant :
i) soit, à hydrolyser le composé de formules (XIIa) ou (XIIb) obtenu ci-dessus à l'étape 1) en milieu acide, pour obtenir un composé de formules (XIIIa) ou (XIIIb) telles que définies précédemment dans lesquelles R⁵ est un atome d'hydrogène, ou
ii) soit, lorsque l'on souhaite obtenir un composé de formule (XIIIa) ou (XIIIb) dans lequel R⁵ est différent d'un atome d'hydrogène, à faire réagir ledit composé de formule (XIIa) ou (XIIb) obtenu ci-dessus à l'étape 1) avec un halogénure de formule (XIV) suivante :
X' -R⁵ (XIV)
dans laquelle X' est un atome d'halogène choisi parmi le chlore et le brome et l'iode et R⁵, a la même signification que celle indiquée ci-dessus pour les composés de formules (XIIIa) et (XIIIb),
pour obtenir un composé de formules (XIIIa) ou (XIIIB) dans lesquelles R⁵ a la même signification que dans la formule (XIV) ci-dessus.

15. Procédé selon la revendication 14, **caractérisé en ce que** le composé de formule (I) est utilisé en une quantité variant de 0,05% mol à 15% molaire.

16. Procédé selon la revendication 14 ou 15, **caractérisé en ce que** la première étape est réalisée à une température variant de 130 à 140°C.

17. Procédé selon l'une quelconque des revendications 14 à 16, **caractérisé en ce que** le solvant organique est choisi parmi le diméthylformamide, le dibutyléther, le diglyme, le triglyme et les xylènes.

18. Procédé selon l'une quelconque des revendications 14 à 17, **caractérisé en ce que** la durée de la première étape varie de 30 min à 20 heures.

19. Procédé selon l'une quelconque des revendications 14 à 18, **caractérisé en ce que** lors de la deuxième étape, l'hydrolyse en milieu acide des composés de formules (XIIIa) ou (XIIIb) est réalisée par ajout, au milieu réactionnel, d'un acide choisi parmi l'acide chlorhydrique, l'acide sulfurique et l'acide trifluoroacétique.

20. Utilisation d'un composé organostannique alcoxylé de formule (I) tel que défini à l'une quelconque des revendications 1 à 5, à titre de catalyseur pour la synthèse, en phase hétérogène, d'un tétrazole choisi parmi le 5-phényl-1*H-*tétrazole, le 5-(4-méthylphényl)-1*H*-tétrazole, le 5-(4-méthoxyphényl)-1*H-*tétrazole, le 5-(3-méthoxyphényl)-1*H-*tétrazole, le 5-(4-nitrophényl)-1*H*-tétrazole, l'acide 4-(1*H*-tetrazol-5-yl)benzoïque, le 5-(3-chlorophényl)-1*H-*tétrazole, le 5-(4-chlorophényl)-1*H-*tétrazole, le 5-(4-hydroxyphényl)-1*H*-tétrazole, le 5-(3-trifluorométhylphényl)-1*H-*tétrazole, le 5-naphtyl-1*H-*tétrazole, le 5-butyl-1*H-*tétrazole, le 5-octyl-1*H-*tétrazole, le 5-(2-bromophényl)-1*H-*tétrazole et le 5-biphényl-2-yl-1*H*-tétrazole.

## Patentansprüche

1. Alkoxylierter Organotinreaktant der folgenden Formel (I): 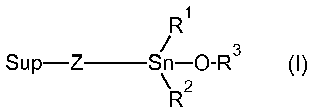 in welcher:
- Sup einen festen makromolekularen Träger darstellt,
- Z einen Verbindungsarm zwischen dem festen makromolekularen Träger Sup und dem Zinnatom darstellt, wobei Z aus den Gruppen der folgenden Formeln (Z-1) bis (Z-6) ausgewählt ist:
-(CH₂)ₙ- (Z-1) 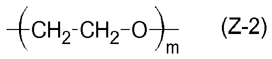 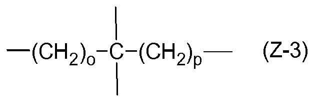 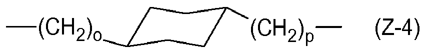 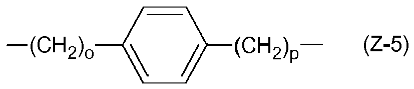
-CH₂-O-(CH₂)_{q}-CH₂-CH₂- (Z-6)
in welchen:
- n und m unabhängig voneinander ganze Zahlen sind, die von 2 bis 24 schwanken,
- o und p unabhängig voneinander ganze Zahlen sind, die von 1 bis 24 schwanken,
- q eine ganze Zahl ist, die von 1 bis 24 schwankt,
- R¹, R² und R³ unabhängig voneinander ein lineares C₁-C₂₄-Alkylradikal, ein verzweigtes oder zyklisches C₃-C₂₄-Alkly-, ein lineares C₂-C₂₄-Alcenyl-, ein verzweigtes oder zyklisches C₃-C₂₄-Alcenyl-, ein Aryl- oder ein Arylalkylradikal darstellen.

2. Reaktant nach Anspruch 1, **dadurch gekennzeichnet, dass** Sup in den organischen Lösungsmitteln unlöslich ist.

3. Reaktant nach Anspruch 1 oder 2, **dadurch gekennzeichnet, dass** das Polymer des festen Trägers aus den Polymeren auf der Basis von Styrol, den Poly(phenylenethern), den Poly(phenylensulfiden) und den Polyamiden ausgewählt ist.

4. Reaktant nach Anspruch 3, **dadurch gekennzeichnet, dass** der Polymerträger aus den Polymerträgern ausgewählt ist, die aus der Copolymerisation des Styrols und des Divinylbenzols als Vernetzungsmittel resultieren.

5. Reaktant nach einem der vorangehenden Ansprüche, **dadurch gekennzeichnet, dass** er aus den Verbindungen ausgewählt ist, in denen:
- Sup ein fester Polymerträger auf der Basis von vernetztem oder nicht vernetztem Styrol ist,
- Z = -(CH₂)ₙ mit n = 3 oder 4 und
- die Radikale R¹, R² und R³ die folgenden Bedeutungen haben:
i) R¹ = R² = R³ = Methyl, Ethyl, Propyl oder Butyl,
ii) R¹ = R² = Methyl und R³ = Ethyl, Propyl oder Butyl,
iii) R¹ = R² = Ethyl und R³ = Methyl, Propyl oder Butyl,
iv) R¹ = R² = Propyl und R³ = Methyl, Ethyl oder Butyl oder
v) R¹ = R² = Butyl und R³ = Methyl, Propyl oder Ethyl.

6. Verfahren für die Herstellung eines alcoxylierten Organotinreaktanten der Formel (I), wie in einem der vorangehenden Ansprüche definiert, **dadurch gekennzeichnet, dass** es mindestens die folgenden Schritte umfasst:
1) einen ersten Schritt des In-Reaktion-Versetzens in einem organischen Lösungsmittel eines festen Trägers der folgenden Formel (II):
Sup-Z-R⁴ (II)
in welcher:
- Sup ein fester makromolekularer Träger ist, wobei der Träger in dem organischen Lösungsmittel unlöslich ist,
- Z einen Verbindungsarm zwischen dem festen makromolekularen Träger Sup und dem Zinnatom darstellt, Z aus den Gruppen der folgenden Formeln (Z-1) bis (Z-6) ausgewählt ist:
-(CH₂)ₙ- (Z-1) 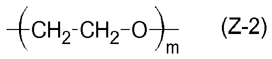 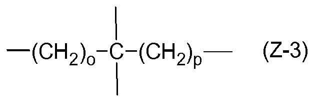 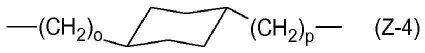 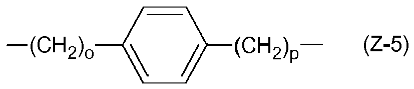
-CH₂-O-(CH₂)_{q}-CH₂-CH₂- (Z-6)
in welchen:
• n und m unabhängig voneinander ganze Zahlen sind, die von 2 bis 24 schwanken,
• o und p unabhängig voneinander ganze Zahlen sind, die von 1 bis 24 schwanken,
• q eine ganze Zahl ist, die von 1 bis 24 schwankt,
- R⁴ Halogenatom oder eine Sulfonatgruppe darstellt, die aus den Mesylat-, Triflat- und Arylsulfonatgruppen ausgewählt ist,
mit einer Organotinverbindung der folgenden Formel (III):
R¹R²SnArM (III)
in welcher:
- R¹ und R² dieselbe Bedeutung haben wie die, die in Anspruch 1 für den Reaktanten der Formel (I) angegeben ist,
- Ar eine substituierte oder nicht substituierte Phenylgruppe ist,
- M ein Metall ist, das aus dem Lithium, dem Natrium, dem Kalium, dem Magnesium und dem Kupfer ausgewählt ist,
um eine geträgerte Organotinverbindung der folgenden Formel (IV) zu erhalten: 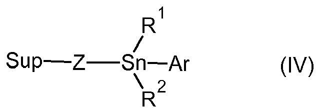 in welcher Sup, Z, R¹, R² und Ar dieselbe Bedeutung haben wie die, die weiter oben für die Verbindungen der Formeln (II) und (III) angegeben ist,
2) einen zweiten Schritt, der darin besteht, die weiter oben nach Abschluss des ersten Schritts erhaltene Verbindung der Formel (IV) in einem organischen Lösungsmittel mit einer Verbindung der Formel X₂, ausgewählt aus I₂, ICI, Cl₂ und Br₂, oder mit einer Bronsted-Säure der Formel HX, in welcher X = Cl⁻, Br⁻, CF₃COO⁻, ArSO₃⁻, oder RCOO- mit R = Alkyl oder Aryl in Reaktion zu versetzen, um eine Verbindung der folgenden Formel (V) zu erhalten: 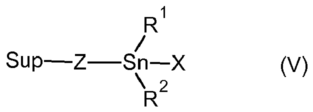 in welcher Sup, Z, R¹, R² und Ar dieselbe Bedeutung haben wie die, die weiter oben für die Verbindungen der Formeln (II) und (III) angegeben ist und X derart ist, wie weiter oben definiert, und
3) einen dritten Schritt, der darin besteht, die weiter oben im zweiten Schritt erhaltene Verbindung der Formel (V) in Anwesenheit einer starken Base in ein hydroalkoholisches Lösungsmittel einzuleiten, das erhaltene Gemisch während einer Dauer von 6 bis 24 Stunden zu rühren, dann ein Karbonat der folgenden Formel (VI) hinzuzufügen: 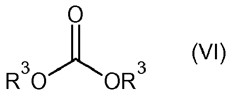 in welchem die Radikale R³ identisch sind und dieselbe Bedeutung haben wie die, die weiter oben in Anspruch 1 für die Reaktanten der Formel (I) angegeben ist, um den erwarteten Reaktanten der Formel (I) zu erhalten.

7. Verfahren nach Anspruch 6, **dadurch gekennzeichnet, dass** der feste Träger der Formel (II) aus den mit Chlorbutylgruppen funktionalisierten Trägern von mit Divinylbenzol vernetztem Polystyrol und ausgewählt ist.

8. Verfahren nach Anspruch 6 oder 7, **dadurch gekennzeichnet, dass** das im ersten Schritt verwendete Lösungsmittel aus dem Tetrahydrofuran, dem 2-Methyl-tetrahydrofuran, dem Dimethoxyethan und dem Dioxan ausgewählt ist.

9. Verfahren nach einem der Ansprüche 6 bis 8, **dadurch gekennzeichnet, dass** der erste Schritt bei einer schrittweise von 0 °C auf 25 °C steigenden Temperatur während einer Dauer von 16 bis 24 Stunden durchgeführt wird.

10. Verfahren nach einem der Ansprüche 6 bis 9, **dadurch gekennzeichnet, dass** der zweite Schritt während bei einer von 40 bis 80 °C schwankenden Temperatur einer Dauer von 12 bis 24 Stunden durchgeführt wird.

11. Verfahren nach einem der Ansprüche 6 bis 10, **dadurch gekennzeichnet, dass** der dritte Schritt bei einer Temperatur zwischen 80 und 140 °C inklusive während einer Dauer von 6 bis 24 Stunden durchgeführt wird.

12. Verwendung einer alcoxylierten Organotinverbindung der Formel (I), so wie in einem der Ansprüche 1 bis 5 definiert, als Katalysator in einem Syntheseverfahren organischer Verbindungen in heterogener Phase.

13. Verwendung nach Anspruch 12 zum Katalysieren der Synthese von 5-substituierten oder 1,5-disubstituierten Tetrazolen.

14. Syntheseverfahren in heterogener Phase von 5-substituierten Tetrazolen der folgenden Formeln (XIIIa) und (XIIIb): 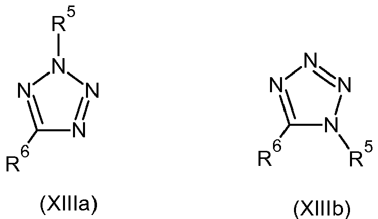 in welchen:
- R⁶ ein lineares C₁-C₂₄-Alkyl-, ein verzweigtes oder zyklisches C₃-C₂₄-Alkly-, ein Aryl-, ein Arylalkyl- oder ein Heteroarylradikal, substituiert oder nicht, darstellt,
- R⁵ ein Wasserstoffatom oder eine Schutzgruppe darstellt, **dadurch gekennzeichnet, dass** das Verfahren umfasst:
1) mindestens einen ersten Schritt, der darin besteht, in einem organischen Lösungsmittel unter inerter Atmosphäre und bei einer Temperatur über oder von gleich 110 °C ein Nitril der folgenden Formel (VIII) in Reaktion zu versetzen:
R⁶-CN (VIII)
in welcher R⁶ dieselbe Bedeutung hat wie die, die weiter oben für die Verbindungen der Formel (XIIIa) und (XIIIb) angegeben ist, mit einem Zinnazids der folgenden Formel (X): 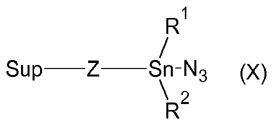 in welcher Sup, Z, R¹ und R² dieselbe Bedeutung haben wie die, die in Anspruch 1 für die Verbindungen der obigen Formel (I) angegeben ist,
wobei der Reaktant der Formel (X) *in situ* durch Reaktion des entsprechenden alcoxylierten Organotinreaktanten der Formel (I), so wie in Anspruch 1 definiert, und eines Trialkylsilylazids der folgenden Formel (IX) erzeugt wird: 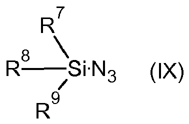 in welcher die identischen oder unterschiedlichen Radikale R⁷, R⁸ und R⁹ ein lineares C₁-C₁₂- (vorzugsweise C₁-C₄-) Alkylradikal oder ein verzweigtes oder zyklisches C₃-C₁₂-Alkylradikal darstellen,
um ein Tetrazol der folgenden Formeln (XIIa) oder (XIIb) zu erhalten: 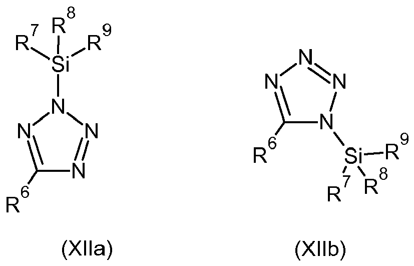 in welchen R⁶ dieselbe Bedeutung wie in der obigen Formel (VIII) hat und R⁷, R⁸ und R⁹ dieselbe Bedeutung wie in der obigen Formel (IX) haben, nach einer Austauschreaktion der Stannyltetrazole der folgenden Formeln (Xla) oder (Xlb): 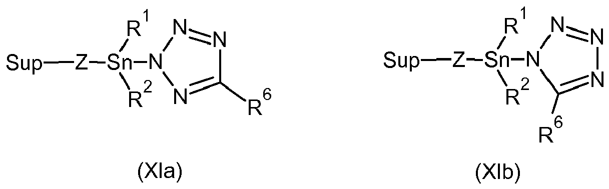 in welchen die Radikale R¹, R² und R⁶ dieselben Bedeutungen haben wie die, die weiter oben für die Verbindungen der jeweiligen Formeln (X) und (VIII) mit der Verbindung der Formel (IX) angegeben sind, und
2) mindestens einen zweiten Schritt, der darin besteht:
i) entweder die in obigem Schritt 1) erhaltene Verbindung der Formeln (XIIa) oder (XIIb) in saurem Milieu zu hydrolysieren, um eine Verbindung der Formeln (XIIIa) oder (XIIIb) zu erhalten, wie zuvor definiert, in welchen R⁵ ein Wasserstoffatom ist,
ii) oder, wenn man eine Verbindung der Formel (XIIIa) oder (XIIIb) erhalten möchte, in welcher sich R⁵ von einem Wasserstoffatom unterscheidet, die Verbindung der im obigen Schritt 1) erhaltene Formel (XIIa) oder (XIIb) mit einem Halogenid der folgenden Formel (XIV) in Reaktion zu versetzen:
X_{'}-R⁵ (XIV)
in welcher X' ein Halogenatom ist, das aus dem Chlor und dem Brom und dem Jod und R⁵ ausgewählt ist, dieselbe Bedeutung hat wie die, die weiter oben für die Verbindungen der Formeln (XIIIa) und (XIIb) angegeben ist,
um eine Verbindung der Formeln (XIIIa) oder (XIIIb) zu erhalten, in welchen R⁵ dieselbe Bedeutung wie in der obigen Formel (XIV) hat.

15. Verfahren nach Anspruch 14, **dadurch gekennzeichnet, dass** die Verbindung der Formel (I) in einer Menge verwendet wird, die von 0,05 % mol bis 15 % mol schwankt.

16. Verfahren nach Anspruch 14 oder 15, **dadurch gekennzeichnet, dass** der erste Schritt bei einer Temperatur durchgeführt wird, die von 130 bis 140 °C schwankt.

17. Verfahren nach einem der Ansprüche 14 bis 16, **dadurch gekennzeichnet, dass** das organische Lösungsmittel aus dem Dimethylformamid, dem Dibutylether, dem Diglym, dem Triglym und den Xylenen ausgewählt ist.

18. Verfahren nach einem der Ansprüche 14 bis 17, **dadurch gekennzeichnet, dass** die Dauer des ersten Schritts von 30 Minuten bis 20 Stunden variiert.

19. Verfahren nach einem der Ansprüche 14 bis 18, **dadurch gekennzeichnet, dass** beim zweiten Schritt die Hydrolyse im sauren Milieu der Verbindungen der Formeln (XIIIa) oder (XIIIb) durch Hinzufügen einer Säure in das Reaktionsmilieu durchgeführt wird, die aus der Salzsäure, der Schwefelsäure und der Trifluoressigsäure ausgewählt ist.

20. Verwendung einer alcoxylierten Organotinverbindung der Formel (I), so wie in einem der Ansprüche 1 bis 5 definiert, als Katalysator für die Synthese in heterogener Phase eines Tetrazols, ausgewählt aus dem 5-Phenyl-1*H*-tetrazol, dem 5-(4-Methylphenyl)-1*H*-tetrazol, dem 5-(4-Methoxyphenyl)-1*H*-tetrazol, dem 5-(3-Methoxyphenyl)-1*H*-tetrazol, dem 5-(4-Nitrophenyl)-1*H*-tetrazol, der 4-(1*H*-Tetrazol-5-yl)benzoesäure, dem 5-(3-Chlorphenyl)-1*H*-tetrazol, dem 5-(4-Chlorphenyl)-1*H-*tetrazol, dem 5-(4-Hydroxyphenyl)-1*H*-tetrazol, dem 5-(3-Trifluormethylphenyl)-1*H-*tetrazol, dem 5-Naphtyl-1*H*-tetrazol, dem 5-Butyl-1*H*-tetrazol, dem 5-Octyl-1H-tetrazol, dem 5-(2-Bromphenyl)-1*H*-tetrazol und dem 5-Biphenyl-2-yl-1*H*-tetrazol.

## Claims

1. An alkoxylated organotin reactant of the following formula (I): 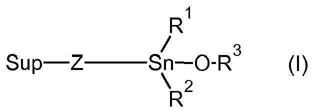 in which:
- Sup represents a macromolecular solid support;
- Z represents a linkage between the macromolecular solid support Sup and the tin atom, Z being selected from the groups of the following formulas (Z-1) to (Z-6):
-(CH₂)ₙ- (Z-1) 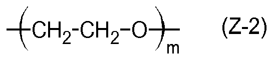 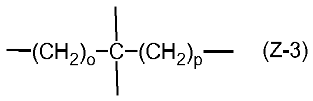 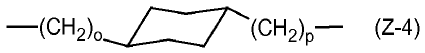 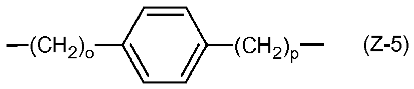
-CH₂-O-(CH₂)_{q}-CH₂-CH₂- (Z-6)
in which:
- n and m, independently of one another, are integers in the range from 2 to 24,
- o and p, independently of one another, are integers in the range from 1 to 24,
- q is an integer in the range from 1 to 24,
- R¹, R² and R³, independently of one another, represent a linear C₁-C₂₄ alkyl radical, a branched or cyclic C₃-C₂₄ alkyl radical, linear C₂-C₂₄ alkenyl, branched or cyclic C₃-C₂₄ alkenyl, aryl or aralkyl.

2. The reactant as claimed in claim 1, **characterized in that** Sup is insoluble in organic solvents.

3. The reactant as claimed in claim 1 or 2, **characterized in that** the polymer of the solid support is selected from styrene-based polymers, poly(phenylene ethers), poly(phenylene sulfides), and polyamides.

4. The reactant as claimed in claim 3, **characterized in that** the polymer support is selected from the polymer supports resulting from the copolymerization of styrene and divinylbenzene as crosslinking agent.

5. The reactant as claimed in any one of the preceding claims, **characterized in that** it is selected from the compounds in which:
- Sup is a polymer solid support based on crosslinked or noncrosslinked styrene;
- Z = -(CH₂)ₙ with n = 3 or 4; and
- the radicals R¹,R², and R³ have the following meanings:
i) R¹ = R² = R³ = methyl, ethyl, propyl or butyl;
ii) R¹ = R² = methyl and R³ = ethyl, propyl or butyl;
iii) R¹ = R² = ethyl and R³ = methyl, propyl or butyl;
iv) R¹ = R² = propyl and R³ = methyl, ethyl or butyl; or
v) R¹ = R² = butyl and R³ = methyl, propyl or ethyl.

6. A method of preparing an alkoxylated organotin reactant of formula (I) as defined in any one of the preceding claims, **characterized in that** it comprises at least the following steps:
1) a first step of reacting, in an organic solvent, a solid support of the following formula (II):
Sup-Z-R4 (II)
in which:
- Sup is a macromolecular solid support, said support being insoluble in said organic solvent;
- Z represents a linkage between the macromolecular solid support Sup and the tin atom, Z being selected from the groups of the following formulas (Z-1) to (Z-6):
-(CH₂)ₙ- (Z-1) 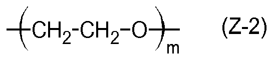 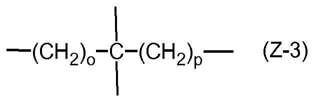 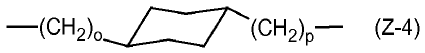 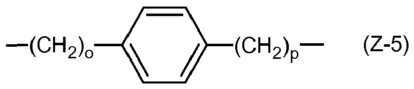
-CH₂-O-(CH₂)_{q}-CH₂-CH₂- (Z-6)
in which:
• n and m, independently of one another, are integers in the range from 2 to 24;
• o and p, independently of one another, are integers in the range from 1 to 24,
• q is an integer in the range from 1 to 24,
- R⁴ represents a halogen atom or a sulfonate group selected from the mesylate, triflate and arylsulfonate groups;
with an organotin compound of the following formula (III):
R¹R²SnArM (III)
in which:
- R¹ and R² have the same meaning as that stated in claim 1 for the reactant of formula (I),
- Ar is a substituted or unsubstituted phenyl group,
- M is a metal selected from lithium, sodium, potassium, magnesium and copper;
to obtain a supported organotin compound of the following formula (IV): 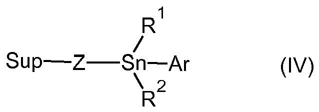 in which Sup, Z, R¹, R² and Ar have the same meaning as that stated above for the compounds of formulas (II) and (III);
2) a second step, consisting of reacting the compound of formula (IV) obtained above at the end of the first step, in an organic solvent, with a compound of formula X₂ selected from I₂, ICl, Cl₂ and Br₂ or with a Bronsted acid of formula HX in which X = Cl⁻, Br⁻, CF₃COO⁻, ArSO₃⁻, or RCOO⁻ with R = alkyl or aryl, to obtain a compound of the following formula (V): 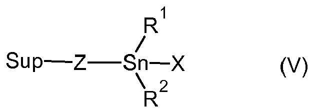 in which Sup, Z, R¹ and R² have the same meaning as that stated above for the compounds of formulas (II) and (III) and X is as defined above; and
3) a third step, consisting of introducing the compound of formula (V) obtained above in the second step in an aqueous-alcoholic solvent in the presence of a strong base, stirring the resultant mixture for a period of from 6 to 24 hours; and then adding a carbonate of the following formula (VI): 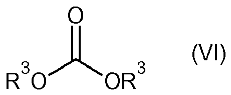 in which the radicals R³ are identical and have the same meaning as that stated above in claim 1 for the reactants of formula (I), to obtain the expected reactant of formula (I).

7. The method as claimed in claim 6, **characterized in that** the solid support of formula (II) is selected from the supports of polystyrene crosslinked with divinylbenzene, and functionalized with chlorobutyl groups.

8. The method as claimed in claim 6 or 7, **characterized in that** the solvent used in the first step is selected from tetralrydrofuran, 2-methyl-tetralrydrofuran, dimethoxyethane, and dioxane.

9. The method as claimed in any one of claims 6 to 8, **characterized in that** the first step is carried out at a temperature gradually increasing from 0°C to 25°C for a time of 16 to 24 hours.

10. The method as claimed in any one of claims 6 to 9, **characterized in that** the second step is carried out at a temperature ranging from 40 to 80°C for 12 to 24 hours.

11. The method as claimed in any one of claims 6 to 10, **characterized in that** the third step is carried out at a temperature between 80 and 140°C for a period of from 6 to 24 hours.

12. The use of an alkoxylated organotin compound of formula (I) as defined in any one of claims 1 to 5, as catalyst, in a method of heterogeneous-phase synthesis of organic compounds.

13. The use as claimed in claim 12, for catalyzing the synthesis of 5-substituted or 1,5-disubstituted tetrazoles.

14. A method of heterogeneous-phase synthesis of 5-substituted tetrazoles of the following formulas (XIIIa) and (XIIIb): 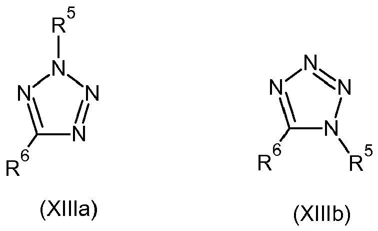 in which:
- R⁶ represents a linear C₁-C₂₄ alkyl, branched or cyclic C₃-C₂₄ alkyl, aryl, aralkyl or heteroaryl radical, substituted or unsubstituted;
- R⁵ represents a hydrogen atom or a protective group, **characterized in that** said method comprises:
1) at least one first step consisting of reacting, in an organic solvent, under inert atmosphere and at a temperature greater than or equal to 110°C, a nitrile of the following formula (VIII):
R⁶-CN (VIII)
in which R⁶ has the same meaning as that stated above for the compounds of formula (XIIIa) and (XIIIb), with a tin nitride of the following formula (X): 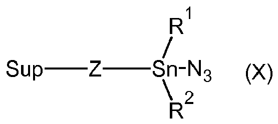 in which Sup, Z, R¹ and R² have the same meaning as that stated in claim 1 for the compounds of formula (I) above,
said reactant of formula (X) being generated in situ by reaction of the corresponding alkoxylated organotin reactant of formula (I) as defined in claim 1 and a trialkylsilyl nitride of the following formula (IX): 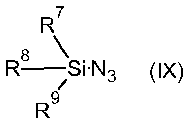 in which the radicals R⁷, R⁸ and R⁹, which may be identical or different, represent a linear C₁-C₁₂ (preferably C₁-C₄) alkyl radical or a branched or cyclic C₃-C₁₂ alkyl radical,
to obtain a tetrazole of the following formula (XIIa) or (XIIb): 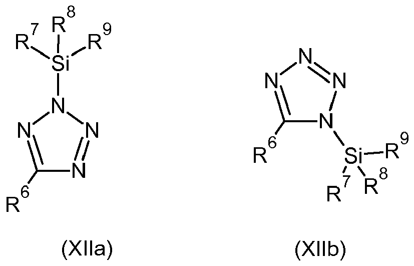 in which R⁶ has the same meaning as in formula (VIII) above and R⁷, R⁸ and R⁹ have the same meaning as in formula (IX) above, after a reaction of exchange of the stannyl tetrazoles of the following formulas (XIa) or (XIb): 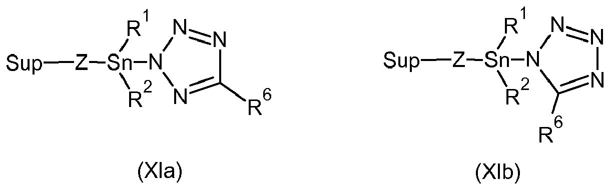 in which the radicals R¹, R² and R⁶ have the same meanings as those stated above for the compounds of formulas (X) and (VIII) respectively, with the compound of formula (IX), and
2) at least one second step consisting:
i) either of hydrolyzing the compound of formulas (XIIa) or (XIIb) obtained above in step 1) in an acid medium, to obtain a compound of formulas (XIIIa) or (XIIIb) as defined above in which R⁵ is a hydrogen atom, or
ii) or, when we wish to obtain a compound of formula (XIIIa) or (XIIIb) in which R⁵ is different from a hydrogen atom, of reacting said compound of formula (XIIa) or (XIIb) obtained above in step 1) with a halide of the following formula (XIV):
X'-R⁵ (XIV)
in which X' is a halogen atom selected from chlorine and bromine and iodine and R⁵ has the same meaning as that stated above for the compounds of formulas (XIIIa) and (XIIIb),
to obtain a compound of formulas (XIIIa) or (XIIIb) in which R⁵ has the same meaning as in formula (XIV) above.

15. The method as claimed in claim 14, **characterized in that** the compound of formula (I) is used in an amount ranging from 0.05 mol% to 15 mol%.

16. The method as claimed in claim 14 or 15, **characterized in that** the first step is carried out at a temperature in the range from 130 to 140°C.

17. The method as claimed in any one of claims 14 to 16, **characterized in that** the organic solvent is selected from dimethylformamide, dibutyl ether, diglyme, triglyme and the xylenes.

18. The method as claimed in any one of claims 14 to 17, **characterized in that** the duration of the first step varies from 30 min to 20 hours.

19. The method as claimed in any one of claims 14 to 18, **characterized in that** during the second step, hydrolysis of the compounds of formulas (XIIIa) or (XIIIb) in an acid medium is carried out by adding an acid selected from hydrochloric acid, sulfuric acid and trifluoroacetic acid to the reaction mixture.

20. The use of an alkoxylated organotin compound of formula (I) as defined in any one of claims 1 to 5, as catalyst for the heterogeneous-phase synthesis of a tetrazole selected from 5-phenyl-1*H-*tetrazole, 5-(4-methylphenyl)-1*H*-tetrazole, 5-(4-methoxyphenyl)-1*H-*tetrazole, 5-(3-methoxyphenyl)-1*H-*tetrazole, 5-(4-nitrophenyl)-1*H-*tetrazole, 4-(1*H-*tetrazol-5-yl)benzoic acid, 5-(3-chlorophenyl)-1*H-*tetrazole, 5-(4-chlorophenyl)-1*H*-tetrazole, 5-(4-hydroxyphenyl)-1*H-*tetrazole, 5-(3-trifluoromethylphenyl)-1*H*-tetrazole, 5-naphthyl-1*H*-tetrazole, 5-butyl-1*H-*tetrazole, 5-octyl-1*H-*tetrazole, 5-(2-bromophenyl)-1*H-*tetrazole and 5-biphenyl-2-yl-1*H-*tetrazole.
